(19) Europäisches Patentamt
European Patent Office
Office européen des brevets

(11) **EP 2 903 693 B1**

(12) **FASCICULE DE BREVET EUROPEEN**

(45) Date de publication et mention
de la délivrance du brevet:
**24.07.2019 Bulletin 2019/30**

(21) Numéro de dépôt: **13782746.5**

(22) Date de dépôt: **24.09.2013**

(51) Int Cl.:
*A61Q 1/00* *(2006.01)*　　　*A61Q 1/10* *(2006.01)*
*A61K 8/81* *(2006.01)*　　　*A61K 8/87* *(2006.01)*
*A61K 8/19* *(2006.01)*　　　*A61K 8/86* *(2006.01)*

(86) Numéro de dépôt international:
**PCT/FR2013/052233**

(87) Numéro de publication internationale:
**WO 2014/053743 (10.04.2014 Gazette 2014/15)**

(54) **COPOLYMERES DANS DES COMPOSITIONS COSMETIQUES**

COPOLYMERE IN KOSMETISCHEN ZUSAMMENSETZUNGEN

COPOLYMERS IN COSMETIC COMPOSITIONS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.10.2012 FR 1202617
02.10.2012 US 201261708733 P
28.06.2013 FR 1356252**

(43) Date de publication de la demande:
**12.08.2015 Bulletin 2015/33**

(73) Titulaire: **COATEX
69730 Genay (FR)**

(72) Inventeurs:
• **SOUZY, Renaud
38430 Saint-Jean de Moirans (FR)**
• **KENSICHER, Yves
F-69620 Theize (FR)**
• **SUAU, Jean-Marc
F-69480 Lucenay (FR)**
• **GUERRET, Olivier
F-46170 Pern (FR)**

(74) Mandataire: **Balmefrezol, Ludovic Francis Pierre
et al
Coatex SAS
35, rue Ampère
69730 Genay (FR)**

(56) Documents cités:
**WO-A1-02/083594　　FR-A1- 2 861 399
FR-A1- 2 900 930　　FR-A1- 2 926 558**

## Description

### DOMAINE DE L'INVENTION

La présente invention concerne le domaine technique de la cosmétique, en particulier le domaine des compositions cosmétiques de revêtement des fibres kératiniques. Plus précisément, la présente invention concerne l'utilisation d'un copolymère particulier pour homogénéiser des compositions comportant des pigments colorants et/ou des nacres et des polymères filmogènes, ainsi que les compositions cosmétiques contenant un tel copolymère.

### ARRIERE PLAN DE L'INVENTION

Les compositions cosmétiques destinées au revêtement des fibres kératiniques dont l'objet est généralement le maquillage et/ou le soin, comportent classiquement des matières colorantes, par exemple des pigments minéraux. En outre, les compositions cosmétiques destinées au revêtement des fibres kératiniques qui doivent résister à l'eau, à la vapeur d'eau, à l'humidité, au sébum et aux larmes, par exemple des formulations de mascaras, comportent généralement un polymère filmogène.

L'homme du métier cherche à contrôler les paramètres rhéologiques, granulométriques, de stabilité et sensoriels lors de la mise en oeuvre, le stockage et la post-utilisation des compositions cosmétiques et plus particulièrement des compositions de revêtements des fibres kératiniques comportant des charges minérales ou organiques, notamment des pigments minéraux. Il utilise pour cela des additifs dispersants ou agents dispersants.

De tels agents dispersants possèdent la capacité de disperser les pigments minéraux, par exemple les pigments d'oxyde métallique, tel que l'oxyde de fer par exemple, dans les compositions les contenant. En effet, on utilise classiquement un agent dispersant pour éviter la floculation des particules et ainsi la perte des propriétés attendues. Concrètement, lorsque l'agent dispersant est introduit dans une formulation cosmétique comprenant des pigments métalliques, il conduit à une viscosité inférieure à celle de la même formulation cosmétique ne contenant pas ledit agent dispersant. On mesure ainsi le pouvoir dispersant d'un agent dans une formulation par une mesure de viscosité. Les agents dispersants permettent en outre la stabilisation des paramètres granulométriques et texturaux des compositions. Ils permettent d'homogénéiser lesdites compositions.

Il existe quatre grandes catégories d'agents dispersants dans les compositions cosmétiques : les huiles, les tensioactifs, les dispersants naturels et les dispersants de type polymérique.

Des additifs de types huiles cosmétiques, par exemple ester, telles que les acides poly(hydroxystéarique), et certain tensioactifs sont par exemple disponibles commercialement pour la dispersion des oxydes de fer dans des formulations cosmétiques pour matières kératiniques. De tels additifs présentent néanmoins l'inconvénient d'impacter négativement l'aspect sensoriel des compositions les contenant et leur utilisation dans de telles compositions sont par conséquent limitées.

Parmi les dispersants naturels ou biosourcés, on peut citer l'utilisation de lignosulfonates et de carbohydrates de type inuline (Abstract of Paper, 244th ACS National Meeting & Exposition, Philadelphia, August 19-23, 2012 Washington DC) et d'acyl-poly(amino acide) et de poly-glutamate (US 20120076840 et JP 2012001503) et des acides poly(aspartiques). De tels dispersants naturels présentent l'inconvénient de nécessiter une purification, afin d'obtenir les masses moléculaires attendues pour une efficacité optimale en tant qu'agent dispersant dans les compositions cosmétiques.

Un certain nombre de documents de l'art antérieur concernent la dispersion des pigments de charge minérale au moyen d'additifs polymériques.

Parmi ceux-ci, on peut citer le document J. Cosmet. Sci., 50, 105-109, 1999 qui concerne de manière générale les additifs pour vernis à ongles à base d'eau et décrit notamment la mise en oeuvre de polymères solubles dans l'eau comme épaississants, stabilisants et dispersants pigmentaires. Des exemples de ces additifs sont les homopolymères de l'oxyde d'éthylène, de l'acide acrylique, de l'acide méthacrylique, de l'alcool polyvinylique et de divers cellulosiques.

On peut également citer l'article Tenside Surf. Det., 36, 1999 qui concerne un domaine technique différent de celui de la présente invention. Ce document décrit l'influence des matériaux polymériques et non-polymériques sur la dispersion des particules d'oxyde de fer dans les eaux utilisées dans les procédés industriels (par exemple : échangeurs de chaleur, systèmes de distillation). Sont notamment cités les polymères suivants : polymères de l'acide acrylique, acide méthacrylique, acide maléïque, acrylamide, 2-acrylamido-2-methylpropane sulfonique acide, et copolymères de l'acide acrylique.

Le brevet WO 2009073384 décrit l'utilisation de copolymères acryliques organosililés. Ces copolymères commercialement disponibles ont un impact non négligeable sur les propriétés sensorielles et organoleptiques des formulations cosmétiques.

En outre, on constate des problèmes d'incompatibilité dans les compositions cosmétiques incorporant des pigments minéraux colorants du type oxyde métallique tel que les oxydes de fer, comprenant des polymères filmogènes et des agents dispersants de type polymérique. La présence simultanée de deux types de polymères au sein de la composition cosmétique est susceptible de conduire dans certains cas à des problèmes d'instabilité, tels que le déphasage ou séparation de phase, l'exsudation, le relargage, et le dépôt ou la sédimentation. Cette incompatibilité est d'autant plus importante qu'on augmente les concentrations en pigments et/ou en polymère filmogène (également appelé agent ou additif filmogène) dans ladite composition cosmétique.

## DESCRIPTION DE L'INVENTION

Un objet de la présente invention est de proposer un additif polymérique qui soit compatible avec les polymères filmogènes utilisés dans les compositions cosmétiques destinées au revêtement des fibres kératiniques résistantes à l'eau, y compris lorsque ces polymères filmogènes sont présents à des concentrations élevées.

Un autre objet de la présente invention est de proposer un additif polymérique qui permette une dispersion efficace des nacres et/ou des pigments colorants utilisés dans de telles compositions, y compris lorsque ces nacres et/ou pigments sont présents à des concentrations élevées.

Un autre objet de la présente invention est de proposer un copolymère qui permette d'homogénéiser les compositions cosmétiques comprenant des polymères filmogènes et plus de 1 % et jusqu'à 20 % en poids de nacre et/ou de pigments colorants, en particulier d'oxyde métallique tel que les oxydes de fer.

Un autre objet de la présente invention est de proposer un copolymère qui facilite la préparation de la composition cosmétique, par exemple en évitant la prise en masse et/ou la floculation.

Un autre objet encore de la présente invention est de proposer une composition cosmétique destinée au revêtement des fibres kératiniques qui soit résistante à l'eau et qui présente une excellente stabilité au stockage.

Les inventeurs se sont rendus compte de manière surprenante qu'en utilisant un copolymère peigne de composition chimique et de masse moléculaire particulières, tous ces objectifs étaient atteints.

L'utilisation d'un tel copolymère permet, en effet, de résoudre les problèmes d'incompatibilités observés avec les polymères filmogènes utilisés dans les compositions cosmétiques du type mascaras résistants à l'eau, et ainsi de permettre à l'homme du métier, lors de la formulation de sa composition cosmétique, de résoudre les problèmes tels que par exemple la prise en masse, la formation d'hétérogénéités irréversibles, la précipitation de pigments lors de l'introduction des différents ingrédients. Un tel copolymère permet ainsi d'homogénéiser les compositions cosmétiques.

L'utilisation d'un tel copolymère permet en outre d'obtenir des compositions cosmétiques qui présentent une meilleure stabilité au stockage, tout en conservant les propriétés organoleptiques attendues.

La présente invention concerne donc l'utilisation d'un copolymère hydrosoluble de type peigne présentant un squelette (ou chaîne principale) et des chaînes latérales polyalkylèneglycol pour homogénéiser des compositions cosmétiques comprenant un polymère filmogène et des nacres et/ou des pigments colorants, en particulier oxyde métallique, tel que oxyde(s) de fer.

Plus précisément, le copolymère hydrosoluble est tel qu'il comporte :

-   au moins un monomère anionique présentant une fonction vinylique polymérisable et un groupement carboxyle, par exemple acide (méth)acrylique ;
-   au moins un monomère présentant une fonction insaturée polymérisable, des unités oxyde d'éthylène (OE) et des unités oxyde de propylène (OP), ces unités (ou motifs) OE et OP pouvant s'enchaîner de manière aléatoire/statistique ou de manière régulière.

En outre, le copolymère selon l'invention présente une masse moléculaire particulière comprise entre 20 000 et 400 000 g/mol, par exemple entre 20 000 et 250 000 g/mol.

Ainsi, un objet de la présente invention concerne l'utilisation d'un copolymère constitué :

a) d'au moins un monomère anionique présentant une fonction vinylique polymérisable et un groupement carboxyle, par exemple l'acide (méth)acrylique et
b) d'au moins un macromonomère de formule (I) :

$$R\text{-}(OP)_m\text{-}(OE)_n\text{-}R'\qquad\qquad(I)$$

-   m et n sont des entiers non nuls et inférieurs à 150,
-   OP et OE désignent respectivement l'oxyde de propylène et l'oxyde d'éthylène et sont disposés en bloc de manière alternée ou statistique,
-   R désigne une fonction insaturée polymérisable,
-   R' représente l'hydrogène ou un groupement alkyle ayant 1, 2, 3 ou 4 atomes de carbone,

ledit copolymère présentant une masse moléculaire comprise entre 20 000 g/mol et 400 000 g/mol, pour homogénéiser une composition cosmétique pour le maquillage et/ou le soin des matières kératiniques comprenant un polymère filmogène et une nacre et/ou un pigment colorant.

Un autre objet de la présente invention concerne une composition cosmétique pour le maquillage et/ou le soin des matières kératiniques, comprenant :

> a) de 1 à 30 % en poids de nacre et/ou pigment colorant,
> b) de 0,1 à 30 % en poids d'un polymère filmogène et
> c) de 0,05 à 8 % en poids d'un copolymère peigne constitué :
>
> > c1) d'au moins un monomère anionique présentant une fonction vinylique polymérisable et un groupement carboxyle, par exemple l'acide (méth)acrylique et
> > c2) d'au moins un macromonomère de formule (I) :

$$R\text{-}(OP)_m\text{-}(OE)_n\text{-}R' \qquad (I)$$

> dans laquelle :
>
> - m et n sont deux entiers non nuls et inférieurs à 150,
> - OP et OE désignent respectivement l'oxyde de propylène et l'oxyde d'éthylène, et sont disposés de manière aléatoire ou de manière régulière,
> - R désigne une fonction insaturée polymérisable,
> - R' représente l'hydrogène ou un groupement alkyle ayant de 1 à 4 atomes de carbone,
>
> ledit copolymère présentant une masse moléculaire comprise entre 20 000 g/mol et 400 000 g/mol.

Un autre objet de la présente invention concerne l'utilisation d'un copolymère selon l'invention pour stabiliser au stockage, tel que défini ci-dessus, une composition cosmétique pour le maquillage et/ou le soin des matières kératiniques comprenant un polymère filmogène et une nacre et/ou un pigment colorant, ce notamment lorsque le pigment colorant et/ou la nacre est présent à une teneur comprise entre 1 et 30 % en poids par rapport au poids total de la composition, et/ou lorsque le polymère filmogène est présent à une teneur comprise entre 0,1 et 30 % en poids par rapport au poids total de la composition.

## DEFINITIONS

Dans le cadre de la présente invention, les extrémités des gammes indiquées (pourcentages en poids, poids moléculaire...) sont inclues dans la portée.

Par « nacre et/ou pigment colorant », on entend au sens large les matières colorantes pulvérulentes. Ces matières colorantes pulvérulentes peuvent être choisies parmi les pigments et les nacres. Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Dans le cadre de la présente invention, le terme « pigment minéral colorant » signifie matières issues de minerais ou obtenues par procédé synthétique, éventuellement modifiées chimiquement, qui une fois mélangées et coformulées dans des compositions cosmétiques permettent d'obtenir des produits cosmétiques de couleurs ou de teintes différentes. Le pigment colorant selon l'invention peut être un pigment minéral synthétique.

Selon un aspect de la présente invention, le pigment colorant est choisi dans le groupe consistant en les oxydes de fer, les oxydes de chrome, les oxydes de zirconium, de zinc ou de cérium, le violet de manganèse, le bleu outremer, l'hydrate de chrome, le noir de carbone et le bleu ferrique.

Selon un autre aspect de la présente invention, le pigment minéral colorant est un oxyde de fer ou un mélange d'oxydes de fer. Les oxydes de fer sont disponibles commercialement ou fabricables à façon.

Par « polymère filmogène », on entend un polymère capable de former à lui seul ou en présence de co-additifs, un film continu et qui adhère aux matières kératiniques. Ce film est de préférence cohésif et isolable si nécessaire. Parmi les polymères filmogènes, pourront être cités les polymères synthétiques se présentant sous forme de dispersions ou d'émulsions de ces dits polymères sous forme d'un latex par exemple.

Les exemples suivants de polymères filmogènes selon l'invention peuvent être cités :

- Les polycondensats : les polyuréthanes, les polyuréthanes acryliques, les polyuréthanes-polyvinylpyrrolidones, les polyesters, les polyesters-polyuréthanes, les polyurées et leurs mélanges.
- Les polymères obtenus par polymérisation radicalaire : les copolymères acryliques et/ou vinyliques obtenus par (co)polymérisation de monomères à insaturation mono et/ou polyéthyléniques tels que les acryliques, les méthacryliques, les esters acryliques, les styréniques, les vinyliques et les mélanges de ceux-ci. On pourra également citer les copolymères acrylique/silicone tels que les copolymères hybrides.

Selon la présente invention, les polymères filmogènes sont différents des copolymères hydrosolubles dispersants objets de la présente invention.

Selon la présente invention, par « stabilité au stockage », on entend une composition qui placée dans une étuve à environ 45°C pendant 3 mois se présente sous une forme identique (ou substantiellement identique) à celle obtenue juste après formulation. En particulier, la composition est dite stable au stockage si elle ne présente aucune des instabilités suivantes : déphasage, séparation de phase, crémage, exsudation, relargage, dépôt ou sédimentation.

Un autre objet de la présente invention concerne l'utilisation d'un copolymère selon l'invention pour stabiliser au stockage, tel que défini ci-dessus, une composition cosmétique pour le maquillage et/ou le soin des matières kératiniques comprenant un polymère filmogène et un pigment minéral colorant, ce notamment lorsque le pigment minéral colorant est présent à une teneur comprise entre 1 et 30 % en poids par rapport au poids total de la composition, et/ou lorsque le polymère filmogène est présent à une teneur comprise entre 0,1 et 30 % en poids par rapport au poids total de la composition.

Par « matières kératiniques », on entend des cils, des sourcils, des poils et/ou des cheveux. Il est également possible d'appliquer la composition selon l'invention sur la peau.

Les « monomères anioniques présentant une fonction vinylique polymérisable et un groupement carboxyle » sont des monomères présentant une charge négative en solution aqueuse basique. Les monomères anioniques présentant une fonction vinylique polymérisable et un groupement carboxyle sont, par exemple, choisis parmi l'acide acrylique et/ou l'acide méthacrylique.

Selon un aspect de la présente invention, le copolymère comprend « au moins un monomère acide (méth)acrylique ». Par « au moins un monomère acide (méth)acrylique », on entend au moins un monomère acide acrylique ou au moins un monomère acide méthacrylique. Ainsi, le copolymère peut comprendre des monomères d'acide acrylique et/ou des monomères d'acide méthacrylique. Ainsi, le squelette acide (méth)acrylique du copolymère selon cet aspect de l'invention peut être exclusivement constitué d'acide acrylique, exclusivement constitué d'acide méthacrylique ou être constitué d'un mélange d'acide acrylique et d'acide méthacrylique.

## DESCRIPTION DETAILLEE DE L'INVENTION

L'état de dispersion des nacres et/ou pigments colorants, en particulier du type oxyde(s) de fer, ainsi que l'abaissement de la viscosité des formulations cosmétiques sont liés à la masse moléculaire, à la composition et à l'architecture des copolymères selon l'invention.

En particulier, les copolymères selon l'invention présentent une masse moléculaire comprise entre 20 000 g/mol et 400 000 g/mol, par exemple entre 20 000 et 250 000 g/mol. Les meilleures mesures de viscosité, et donc le meilleur effet sur l'homogénéité des compositions, sont en effet obtenus pour des compositions cosmétiques comportant les copolymères de masses moléculaires moyennes sélectionnées dans cette gamme.

Dans le cadre de la présente invention, par « masse moléculaire », on entend masse moléculaire moyenne en poids ou $M_w$. La masse moléculaire est déterminée par chromatographie d'exclusion stérique (SEC), encore appelée chromatographie par perméation de gel (GPC).

Selon un mode de réalisation de la présente invention, le copolymère présente une masse moléculaire comprise entre 20 000 g/mol et 350 000 g/mol, par exemple entre 30 000 g/mol et 380 000 g/mol ou entre 40 000 g/mol et 250 000 g/mol.

Selon un autre mode de réalisation de la présente invention, le copolymère présente une masse moléculaire comprise entre 50 000 g/mol et 110 000 g/mol.

Selon un mode de réalisation de la présente invention, le copolymère est tel que R dans le macromonomère de formule (I) représente la fonction méthacrylate, la fonction méthacryluréthane, la fonction acrylate, la fonction vinyle, la fonction méthallyle ou la fonction allyle.

Ledit copolymère selon l'invention est obtenu par des procédés connus de copolymérisation radicalaire conventionnelle en solution, en émulsion directe ou inverse, en bulk, en suspension ou précipitation dans des solvants appropriés, en présence d'initiateurs et d'agents de transfert connus, ou encore par des procédés de polymérisation radicalaire contrôlée tels que la méthode dénommée Réversible Addition Fragmentation Transfer (RAFT), la méthode dénommée Atom Transfer Radical Polymerization (ATRP), la méthode dénommée Nitroxide Mediated Polymerization (NMP) ou encore la méthode dénommée Cobaloxime Mediated Free Radical Polymerization.

Selon un aspect de la présente invention, le copolymère comprend également un ester de l'acide (méth)acrylique, par exemple l'acrylate d'éthyle.

Selon un aspect de la présente invention, le copolymère est un copolymère (méth)acrylique et est constitué, exprimé en pourcentage en poids de chacun de ses constituants, de :

a) 5 % à 30 % d'au moins un monomère acide (méth)acrylique,
b) 70 % à 95 % d'au moins un macromonomère de formule (I),
c) 0 % à 20 % d'au moins un monomère qui est un ester de l'acide (méth)acrylique,

la somme des pourcentages a), b) et c) étant égale à 100 %.

Selon un autre aspect de la présente invention, le copolymère est un copolymère (méth)acrylique et est constitué, exprimé en pourcentage en poids de chacun de ses constituants, de :

a) 5 % à 15 % d'au moins un monomère acide (méth)acrylique,
b) 85 % à 95 % d'au moins un macromonomère de formule (I),
c) 0 % à 10 % d'au moins un monomère qui est un ester de l'acide (méth)acrylique,

la somme des pourcentages a), b) et c) étant égale à 100 %.

Selon un aspect de la présente invention, le copolymère est tel que dans la formule (I), m et n sont compris entre 10 et 90.

Selon l'invention, ledit copolymère peut être totalement ou partiellement neutralisé par un ou plusieurs agents de neutralisation disposant d'un cation monovalent ou polyvalent. Lesdits agents peuvent par exemple être choisis dans le groupe consistant en l'ammoniaque, les hydroxydes et/ou oxydes de calcium, de magnésium, les hydroxydes de sodium, de potassium, de lithium, les amines primaires, secondaires ou tertiaires aliphatiques et/ou cycliques comme par exemple la stéarylamine, les éthanolamines (mono-, di-, triéthanolamine), la mono et diéthylamine, la cyclohexylamine, la méthylcyclohexylamine, l'amino méthyl propanol, la morpholine.

Le copolymère selon l'invention présente une fonction dispersante dans les compositions cosmétiques contenant des nacres et/ou des pigments colorants tels que les oxydes métalliques, ce, y compris à des concentrations pigmentaires volumiques (CPV) élevées.

Selon un mode de réalisation de la présente invention, la composition cosmétique selon l'invention présente une concentration pigmentaire volumique notamment comprise entre 15 et 50%.

La « concentration pigmentaire volumique » est définie par la formule suivante :

$$\text{CPV } (\%) = 100 \text{ x } V_c / (V_c + V_l)$$

avec $V_c$ qui représente le volume des pigments colorants et
$V_1$ qui représente le volume sec du polymère filmogène dans la composition cosmétique.

Selon un aspect de la présente invention, la composition cosmétique comprend de 1 à 30 % en poids de nacre et/ou de pigment colorant, par exemple de 1 à 20 % en poids ou de 1 à 10 % en poids (les extrémités des gammes de % sont incluses dans la portée).

Selon un autre aspect de la présente invention, la composition cosmétique comprend de 3 à 30 % en poids de nacre et/ou de pigment colorant, par exemple de 3 à 20 % en poids ou de 3 à 10 % en poids.

Selon un autre aspect de la présente invention, la composition cosmétique comprend de 0,1 à 20 % en poids d'un polymère filmogène, par exemple de 0,1 à 10 % en poids.

Selon un mode de réalisation de la présente invention, la composition cosmétique pour le maquillage et/ou le soin des matières kératiniques, comprend :

a) de 3 à 25 % en poids de nacre et/ou pigment colorant,
b) de 8 à 15 % en poids d'un polymère filmogène et
c) de 0,05 à 8 % en poids d'un copolymère peigne constitué :

c1) d'au moins un monomère anionique présentant une fonction vinylique polymérisable et un groupement carboxyle, par exemple l'acide (méth)acrylique et

c2) d'au moins un macromonomère de formule (I) :

$$R\text{-}(OP)_m\text{-}(OE)_n\text{-}R' \qquad (I)$$

dans laquelle:

- m et n sont deux entiers non nuls et inférieurs à 150,
- OP et OE désignent respectivement l'oxyde de propylène et l'oxyde d'éthylène, et sont disposés de manière aléatoire ou de manière régulière,
- R désigne une fonction insaturée polymérisable,
- R' représente l'hydrogène ou un groupement alkyle ayant de 1 à 4 atomes de carbone,

ledit copolymère présentant une masse moléculaire comprise entre 20 000 g/mol et 400 000 g/mol.

La composition cosmétique selon l'invention peut se présenter sous forme de phase continue aqueuse ou sous forme anhydre ou sous forme d'émulsion eau-dans-huile, huile-dans-eau ou dispersion eau-dans-huile ou huile-dans-eau. Elle peut être solide, liquide ou pâteuse. La composition selon l'invention peut ainsi comprendre une phase aqueuse qui peut être constituée essentiellement d'eau.

Selon un aspect de la présente invention, ces compositions cosmétiques sont constituées d'au moins deux phases, à savoir une phase aqueuse et une phase non aqueuse, lesdites phases pouvant elles-mêmes incorporer des particules solides telles que les pigments colorants et/ou les nacres. Le copolymère selon la présente invention est généralement incorporé dans la phase aqueuse de la composition cosmétique, mais il peut l'être dans la phase non aqueuse.

Ainsi, selon un aspect de la présente invention la composition cosmétique comprend, par rapport au poids total de la composition, de :

a) 10 % à 99,9 % en poids de phase aqueuse,

b) 0,1 % à 90 % en poids total de phase non aqueuse,

la somme a) + b) étant égale à 100 %.

Selon un autre aspect de la présente invention la composition cosmétique comprend, par rapport au poids total de la composition, de :

a) 15 % à 99,5 % en poids de phase aqueuse,

b) 0,5 % à 85 % en poids total de phase non aqueuse,

la somme a) + b) étant égale à 100 %.

Selon un autre aspect encore de la présente invention la composition cosmétique comprend, par rapport au poids total de la composition, de :

a) 50 % à 70 % en poids de phase aqueuse,

b) 30 % à 50 % en poids total de phase non aqueuse,

la somme a) + b) étant égale à 100 %.

La phase aqueuse de la composition peut être constituée d'un mélange d'eau et de solvants organiques miscibles à l'eau (miscibilité dans l'eau supérieure à 50 % en poids à 25°C). Ces solvants sont par exemple choisis parmi les monoalcools inférieurs comportant de 1 à 5 atomes de carbone tels que l'éthanol, l'isopropanol, les glycols comportant de 2 à 8 atomes de carbone tels que le propylène glycol, l'éthylène glycol, le 1,3-butylène glycol, le dipropylène glycol, les cétones en C3-C4, les aldéhydes en C2-C4 et les alcools éthoxylés.

La phase non aqueuse (ou phase grasse) de la composition cosmétique selon la présente invention est susceptible de comprendre des ingrédients naturels ou synthétiques non miscibles à l'eau, liquides à température ambiante (25°C) et/ou solides à température ambiante, pouvant notamment être choisis dans le groupe consistant en les cires, les huiles, les corps gras pâteux, les gommes et leurs mélanges.

Par « cire », dans le cadre de la présente invention, on entend un composé lipophile, solide à température ambiante (25°C), déformable ou non, à changement d'état solide/liquide réversible, ayant un point de fusion supérieur ou égal à 30°C pouvant aller jusqu'à 200°C et notamment jusqu'à 120°C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange. En particulier, les cires convenant à l'invention peuvent présenter un point de fusion supérieur ou égal à 45°C, et en particulier supérieur ou égal à 55°C.

Par « huile », on entend un corps gras liquide à température ambiante (25°C). L'huile peut être volatile ou non volatile. Une huile volatile est susceptible de s'évaporer au contact de la peau ou de la fibre kératinique en moins d'une heure, à température ambiante et pression atmosphérique. Une huile non volatile est une huile restant sur la peau ou la fibre kératinique à température ambiante et pression atmosphérique au moins plusieurs heures. L'huile peut être choisie parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges. Plus précisément, par huile hydrocarbonée, on entend une huile comportant principalement des atomes de carbone et d'hydrogène et éventuellement une ou plusieurs fonctions choisies parmi les fonctions hydroxyle, ester, éther, carboxylique. Des huiles volatiles et des huiles non volatiles sont disponibles commercialement.

La composition selon l'invention peut comprendre une teneur en huiles, volatiles ou non volatiles, et/ou en cire allant de 1 à 80 % en poids par rapport au poids total de la composition, par exemple de 5 à 70 % en poids, ou de 10 à 50 % ou encore de 15 à 40 % en poids.

Selon un autre aspect de la présente invention, la composition cosmétique comprend de 0,05 à 5 % en poids dudit copolymère en l'état, par exemple de 0,1 à 5 % en poids ou par exemple de 0,1 à 3 % en poids.

La composition de l'invention peut comprendre, en outre, tout additif usuellement utilisé en cosmétique.

Ainsi, la composition cosmétique selon l'invention est, de plus, susceptible de contenir un ou plusieurs des ingrédients suivants :

- des particules réfléchissantes,
- des agents tensioactifs ou émulsionnants ou émulsifiants,
- des émollients,
- des humectants, des hydratants,
- des agents neutralisants, alcalinisants ou acidifiants,
- des conservateurs,
- des charges,
- des antioxydants,
- des parfums,
- les plastifiants,
- des actifs cosmétiques comme par exemple des vitamines,
- des filtres solaires.

Ces additifs peuvent être présents dans la composition en une teneur allant de 0,01 à 30 %, du poids total de la composition.

Par exemple, la composition est susceptible de comprendre au moins un autre agent structurant choisi parmi les agents épaississants, les gélifiants lipophiles conventionnellement utilisés en cosmétique et leurs mélanges.

A titre de gélifiants lipophiles conventionnellement utilisés en cosmétique, on peut citer par exemple les gélifiants lipophiles minéraux tels que les argiles ou les silices, les gélifiants lipophiles organiques polymériques tels que les organopolysiloxanes élastomériques partiellement ou totalement réticulés, les copolymères séquencés du type polystyrène/copoly(éthylène-propylène), les polyamides et leurs mélanges. Quant à la phase aqueuse de la composition, elle peut être épaissie par un agent épaississant. Parmi les agents épaississants de phase aqueuse utilisables selon l'invention, on peut citer les épaississants cellulosiques, les argiles, les polysaccharides, les polymères acryliques, les polymères associatifs et leurs mélanges. Comme agent épaississant hydrophile, on peut citer en particulier les copolymères AMPS/acrylamide de type SEPIGEL ou SIMULGEL (SEPPIC).

Dans la composition selon l'invention, la teneur en agent épaississant de phase aqueuse peut aller de 0,1 % à 15 % en poids, par rapport au poids total de la composition, par exemple de 1 % à 10 %, ou par exemple de 1 à 5 % en poids.

La composition peut également comprendre des agents tensioactifs non ioniques, anioniques, cationiques, amphotériques ou encore des émulsionnants tensioactifs.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Les charges peuvent être choisies parmi celles bien connues de l'homme du métier et couramment utilisées dans les compositions cosmétiques.

La composition cosmétique pour le maquillage et/ou le soin des matières kératiniques selon l'invention peut se présenter sous la forme d'un produit pour les cils, sous la forme d'un produit pour les sourcils ou sous la forme d'un produit de maquillage des cheveux ou des poils, plus spécialement sous la forme d'un mascara. Elle peut se présenter sous forme d'un fluide, sous forme solide ou sous forme biphasique. Elle peut par exemple se trouver sous forme de stick ou sous forme d'une pâte souple.

Cette composition cosmétique peut être sur («Top Coat») ou sous («Base Coat») le maquillage.

## EXEMPLES

Dans chacun des exemples suivants, la masse moléculaire des copolymères selon l'invention est déterminée par chromatographie d'exclusion stérique (CES) ou en anglais « Gel Permeation Chromatography » (GPC).

Une telle technique met en oeuvre un appareil de chromatographie liquide de marque WATERS™ doté de deux détecteurs. L'un de ces détecteurs combine la diffusion dynamique statique de la lumière à un angle de 90°C à la viscosimétrie mesurée par un viscosimètre détecteur VISCOTEK™ MALVERN™. L'autre de ces détecteur est un détecteur de concentration réfractométrique de marque WATERS™.

Cet appareillage de chromatographie liquide est doté de colonnes d'exclusion stérique convenablement choisies par l'homme du métier afin de séparer les différents poids moléculaires des polymères étudiés. La phase liquide d'élution est une phase aqueuse contenant 1 % de $KNO_3$. De manière détaillée, selon une première étape, on dilue à 0,9 % sec la solution de polymérisation dans l'éluant de la CES, qui est une solution à 1 % de $KNO_3$. Puis, on filtre à 0,2 $\mu$m. 100 $\mu$L sont ensuite injectés dans l'appareil de chromatographie (éluant : une solution à 1 % de $KNO_3$).

L'appareil de chromatographie liquide contient une pompe isocratique (WATERS 515) dont le débit est réglé à 0,8 ml/min. L'appareil de chromatographie comprend également un four qui lui-même comprend en série le système de colonnes suivant : une précolonne de type GUARD COLUMN ULTRAHYDROGEL WATERS™ de 6 cm de long et 40 mm de diamètre intérieur, une colonne linéaire de type ULTRAHYDROGEL WATERS™ de 30 cm de long et 7,8 mm de diamètre intérieur et deux colonnes ULTRAHYDROGEL 120 ANGSTROM WATERS™ de 30 cm de longueur et 7,8 mm de diamètre intérieur. Le système de détection quant à lui se compose d'une part d'un détecteur réfractométrique de type RI WATERS™ 410 et de l'autre côté d'un double détecteur viscosimètre et diffusion de la lumière à un angle de 90° de type 270 DUAL DETECTOR MALVERN™. Le four est porté à la température de 55°C, et le réfractomètre est porté à la température de 45°C.

L'appareil de chromatographie est étalonné par un unique étalon de PEO 19k de type PolyCAL™ MALVERN™.

### Exemple 1

On prépare des copolymères de la présente invention selon les procédés décrits ci-dessous.

### Essai 1-1

Dans un réacteur de 1 litre muni d'une agitation mécanique et d'un chauffage de type bain d'huile, on pèse :

- 514 g d'eau,
- 27 g d'acide méthacrylique,
- 217 g d'un macromonomère qui est un méthacrylate de poly(alkylene glycol) de masse moléculaire 3000 et constitué de 70 % en masse de poly(oxyde d'éthylène) et de 30 % en masse de poly(oxyde de propylène) et
- 23 g d'eau.

Dans un premier récipient, on pèse 1 g de 1,8-dimercapto-3,6-dioxaoctane (DMDO).

Dans un deuxième récipient, on pèse 1,54 g de persulfate d'ammonium et 5 g d'eau déionisée. On introduit à 65°C et successivement en une fois les contenus du récipient 1 puis 2.

On cuit alors 3 heures à 65°C.

L'ensemble est alors neutralisé avec de la soude 50 % jusqu'à pH = 7 et dilué pour atteindre une concentration de 25 % en matières sèches.

Le polymère final possède la caractéristique suivante : Mw = 350 000 g/mol.

### Essai 1-2

Dans un réacteur de 1 litre muni d'une agitation mécanique et d'un chauffage de type bain d'huile, on pèse 284 g d'eau.

Dans un premier récipient, on pèse :

- 48,7 g d'acide méthacrylique,
- 339,5 g d'un macromonomère qui est un méthacrylate de poly(alkylène glycol) de masse moléculaire 3 000 et constitué de 70 % en masse de poly(oxyde d'éthylène) et de 30 % en masse de poly(oxyde de propylène), et
- 25,4 g d'eau.

Dans un deuxième récipient, on pèse 2,44 g de 1,8-dimercapto-3,6-dioxaoctane (DMDO).

Dans un troisième récipient une solution constituée de 1,44 g de persulfate d'ammonium et 80 g d'eau déionisée.

On introduit à 65°C et en 3 heures les ingrédients des 3 récipients dans le réacteur chauffé et agité.

On cuit alors une heure supplémentaire à 67°C.

L'ensemble est alors neutralisé avec de la soude 50 % jusqu'à pH = 7 et dilué pour atteindre une concentration de 40 % en matières sèches.

Le polymère final possède la caractéristique suivante : Mw = 71000 g/mol.

**Essai 1-3**

Dans un réacteur de 1 litre muni d'une agitation mécanique et d'un chauffage de type bain d'huile, on pèse 257 g d'eau.

Dans un premier récipient, on pèse :

- 50,0 g d'acide acrylique,
- 339,5 g d'un macromonomère qui est un méthacrylate de poly(alkylène glycol) de masse moléculaire 3 000 et constitué de 70 % en masse de poly(oxyde d'éthylène) et de 30 % en masse de poly(oxyde de propylène), et
- 21 g d'eau.

Dans un deuxième récipient, on pèse 4,45 g de 1,8-dimercapto-3,6-dioxaoctane (DMDO).

Dans un troisième récipient, on prépare une solution constituée de 3,6 g de persulfate d'ammonium et 80 g d'eau déionisée.

On introduit à 65°C et en 3 heures les ingrédients contenus dans les 3 récipients dans le réacteur chauffé et agité.

On cuit alors une heure supplémentaire à 67°C.

L'ensemble est alors neutralisé avec de la soude 50 % jusqu'à pH = 7 et dilué pour atteindre une concentration de 40 % en matières sèches.

Le polymère final possède la caractéristique suivante : Mw = 45 000 g/mol.

L'ensemble des exemples 1 à 5 qui suivent illustrent l'utilisation des copolymères selon les essais 1-1 à 1-3 dans différentes formulations de composition de mascara. Les valeurs mentionnées dans la dernière colonne des tableaux indiquent les masses en grammes.

Les résultats (non montrés) indiquent par ailleurs que la double sélection opérée sur le choix du macromonomère d'une part, et sur la masse moléculaire du copolymère d'autre part, conduisent à des compositions homogènes caractérisées par une diminution de la viscosité qui ne présentent pas de difficultés (formation d'hétérogénéités, précipitation, déplétion...) lors de l'élaboration du mascara, ainsi qu'une stabilité au stockage. Ainsi, on déduit un très bon niveau de compatibilité entre le polymère de l'invention et le polymère filmogène utilisé dans une formulation cosmétique qui comprend différents oxydes de fer noir ou des pigments organiques.

**Exemple 2**

**Tableau 1**

|  | 2-1 | 2-2 |
|---|---|---|
|  | Art antérieur | Invention |
| Phase A | | |
| Eau déionisée | qsp 100 | qsp 100 |
| **Oxyde de fer noir (BK 5000 HP - Mineral and Pigment Solutions Inc.)** | 7 | 7 |
| **Polymère Essai 1-1 (quantité en l'état)** | 0 | **0.1** |

(suite)

| | 2-1 | 2-2 |
|---|---|---|
| | Art antérieur | Invention |
| Phase A | | |
| Conservateur | 0.1 | 0.1 |
| Antimousse | 0.1 | 0.1 |
| PEG-200 Glycéryl stéarate (Simulsol 220 - Seppic) | 4 | 4 |
| | | |
| Phase B | | |
| Cire d'abeille | 7.4 | 7.4 |
| Cire de carnauba | 3.5 | 3.5 |
| | | |
| Phase C | | |
| Copolymère acrylamide / acrylamido-2-méthylpropane sulfonate de sodium en émulsion inverse dans l'isohexadécane (Simulgel 600 - Seppic) | 3.5 | 3.5 |
| Dispersion aqueuse de polyuréthane (polycaprolactone/4,4' diphényle méthane diisocyanate) (Disperbond D31W40 - Merquinsa) - Polymère filmogène | 8 | 8 |

Les composés de la phase B sont fondus à 98°C puis homogénéisés sous agitation. L'oxyde de fer noir est dispersé dans l'eau en présence des autres composés de la phase A. La phase A homogène est ensuite chauffée sous agitation modérée jusqu'à 93°C puis incorporée sous forte agitation dans la phase B. Le mélange est effectué pendant 5 minutes puis sa température est abaissée à 40°C. La phase C est alors incorporée sous agitation modérée.

Les essais sont alors refroidis à température ambiante sous agitation modérée puis conditionnés en flacons.

Un échantillon de cheveux blond propres, homogène en couleur, est partagé en 2 pour réaliser les essais de mascara.

Les essais sont utilisés pour enduire soigneusement les cheveux. Après enduction, les cheveux sont séchés au sèche-cheveux à 65°C. La coloration des mèches réalisées avec les 2 essais est mesurée au colorimètre de surface. De la mesure L.a.b., la valeur L est utilisée pour déterminer l'intensité de couleur de chaque essai. L'essai avec le polymère de l'invention (exemple 2-2) montre une intensité de couleur de 9 % supérieure à l'essai de l'art antérieur (exemple 2-1).

## Exemple 3

**Tableau 2**

| | 3-1 | 3-2 |
|---|---|---|
| | Art antérieur | Invention |
| Phase A | | |
| Eau déionisée | qsp 100 | qsp 100 |
| **Oxyde de fer rouge (CG160 - Mineral and Pigment Solutions Inc.)** | 6 | 6 |
| **Polymère Essai 1-2 (quantité en l'état)** | 0 | **0.1** |
| Conservateur | 0.1 | 0.1 |
| Ethanol | 10 | 10 |
| Propylène glycol | 2 | 2 |
| | | |

(suite)

| Phase B | | |
|---|---|---|
| Eau désionisée | 30 | 30 |
| Alcool polyvinylique (Selvol PVA 125 - Sekisui) | 1 | 1 |
| Gomme arabique | 3 | 3 |
| Hydroxyéthyl cellulose (Cellosize HEC QP 300 - Amerchol) | 1.5 | 1.5 |
| Polystyrène sulfonate de sodium (Flexan II - Akzo Nobel) | 1 | 1 |
| **Copolymère styrène / acrylate (Joncryl 77 - BASF) - polymère filmogène** | 10 | 10 |
| | | |
| Phase C | | |
| Eau désionisée | 11.25 | 11.25 |
| Cire de Carnauba | 5.4 | 5.4 |
| PEG-30 Glycéryl Stéarate (Tagat S - Goldschmidt) | 1.35 | 1.35 |
| Ethanol | 2 | 2 |

Le PVA, la gomme arabique et l'hydroxyéthyl cellulose sont dispersés puis dissous dans l'eau de la phase B.

Après dissolution, les autres composés de la phase B sont ajoutés et mélangés.

La cire et le tensioactif de la phase C sont portés à 90°C puis homogénéisés.

L'eau de la phase C, préalablement chauffée à 90°C est alors incorporée au mélange cire et tensioactif puis le tout est soigneusement mélangé pendant 10 minutes, passé ce temps, le mélange est refroidi à 40°C puis l'éthanol est ajouté et après mélange, la phase C est refroidie à température ambiante.

Les composés de la phase A sont mélangés pour disperser l'oxyde de fer rouge.

Sur la phase A ainsi réalisée, on ajoute sous agitation la phase C puis la phase B.

Le mélange est homogénéisé pendant 10 minutes puis conditionné en flacon.

Un échantillon de cheveux blonds propres, homogène en couleur, est partagé en 2 pour réaliser les essais de mascara.

Les essais sont utilisés pour enduire soigneusement les cheveux.

Après enduction, les cheveux sont séchés au sèche-cheveux à 40°C.

La coloration des mèches réalisées avec les 2 essais est mesurée au colorimètre de surface.

De la mesure L.a.b., la valeur L est utilisée pour déterminer l'intensité de couleur de chaque essai.

L'essai avec le polymère de l'invention (exemple 3-2) montre une intensité de couleur de 12 % supérieure à l'essai de l'art antérieur (exemple 3-1).

## Exemple 4

**Tableau 3**

| | 4-1 | 4-2 |
|---|---|---|
| | Art antérieur | Invention |
| Phase A | | |
| Eau déionisée | 5 | 5 |
| **Oxyde de fer noir (BK 5000 HP - Mineral and Pigment Solutions Inc.)** | 3 | 3 |
| **Oxyde de fer jaune (PURICOLOR Yellow PYE42 - BASF)** | 1 | 1 |
| **Polymère Essai 1-2 (quantité en l'état)** | 0 | **0.1** |
| Conservateur | 0.1 | 0.1 |
| Carbonate de propylène | 2 | 2 |

(suite)

|  | 4-1 | 4-2 |
|---|---|---|
|  | Art antérieur | Invention |
| Phase A | | |
|  | | |
| Phase B | | |
| Cire de paraffine | 2.5 | 2.5 |
| Cire de Carnauba | 6 | 6 |
| Cire d'abeille | 5 | 5 |
| Hydroxystéaryloxy stéarate d'alkyle C20-C40 (Kester Wax K82P - Koster Keunen) | 3.5 | 3.5 |
| Cire de polyéthylène | 2 | 2 |
| Polylaurate de vinyle (Mexomère PP - Chimex) | 0.75 | 0.75 |
| **Copolymère acétate de vinyle / stéarate d'allyle (Mexomère PQ - Chimex) - Polymère filmogène** | 2.2 | 2.2 |
| Hectorite modifiée (Bentone 27V - Elementis) | 5.5 | 5.5 |
|  | | |
| Phase C | | |
| Isododécane | qsp 100 | qsp 100 |

Les composés de la phase B sont fondus à 98°C puis homogénéisés sous agitation. Les oxydes de fer noir et jaune sont dispersés dans l'eau en présence des autres composés de la phase A. La phase A homogène est ensuite chauffée sous agitation modérée jusqu'à 93°C puis incorporée sous forte agitation dans la phase B. le mélange est effectué pendant 5 minutes puis sa température est abaissée à 70°C. La phase C est alors incorporée sous agitation modérée. Les essais sont alors refroidis à température ambiante sous agitation modérée puis conditionnés en flacons.

Un échantillon de cheveux blond propres, homogène en couleur, est partagé en 2 pour réaliser les essais de mascara.

Les essais sont utilisés pour enduire soigneusement les cheveux. Après enduction, les cheveux sont séchés au sèche-cheveux à 65°C. La coloration des mèches réalisées avec les 2 essais est mesurée au colorimètre de surface. De la mesure L.a.b., la valeur L est utilisée pour déterminer l'intensité de couleur de chaque essai. L'essai avec le polymère de l'invention (exemple 4-2) montre une intensité de couleur de 14 % supérieure à l'essai de l'art antérieur (exemple 4-1).

**Exemple 5**

**Tableau 4**

|  | 5-1 | 5-2 |
|---|---|---|
|  | Art antérieur | Invention |
| Phase A | | |
| Eau déionisée | qsp 100 | qsp 100 |
| **Pigment organique Vert (Vibracolor Green PGR7 - BASF)** | 0.5 | 0.5 |
| **Pigment organique Bleu (Vibracolor Blue PBL 15:3-L - BASF)** | 4.5 | 4.5 |
| **Polymère Essai 1-3 (quantité en l'état)** | **0** | **0.1** |
| Conservateur | 0.1 | 0.1 |
| Siméthicone (antimousse) | 0.4 | 0.4 |

(suite)

|  | 5-1 | 5-2 |
|---|---|---|
|  | Art antérieur | Invention |
| Phase A | | |
|  | | |
| Phase B | | |
| Cire de Candelilla - Liant | 24 | 24 |
| **Copolymère Vinylpyrrolidone - Eicosène (Ganex V220 - Ashland) - polymère filmogène** | 1 | 1 |
| Sulfonate d'alkyle secondaire C14-C17 (Hostapur SAS - Clariant) | 5 | 5 |
|  | | |
| Phase C | | |
| Hydroxyéthyl cellulose (Cellosize HEC QP 300 - Amerchol) | 0.9 | 0.9 |

La cire de Candelilla de la phase B est fondue à 80°C puis le copolymère VP-Eicosène et l'Hostapur SAS sont introduit et mélangé sous agitation. Les pigments organiques bleu et vert sont dispersés dans l'eau en présence des autres composés de la phase A. La phase A homogène est ensuite chauffée sous agitation modérée jusqu'à 75°C puis incorporée sous forte agitation dans la phase B. le mélange est effectué pendant 5 minutes puis sa température est abaissée à 40°C. La phase C est alors incorporée sous agitation modérée et mélangée pendant 20 minutes. Les essais sont alors refroidis à température ambiante sous agitation modérée puis conditionnés en flacons.
Un échantillon de cheveux blonds propres, homogène en couleur, est partagé en 2 pour réaliser les essais de mascara.
Les essais sont utilisés pour enduire soigneusement les cheveux. Après enduction, les cheveux sont séchés au sèche-cheveux à 65°C. La coloration des mèches réalisées avec les 2 essais est mesurée au colorimètre de surface. De la mesure L.a.b., la valeur L est utilisée pour déterminer l'intensité de couleur de chaque essai. L'essai avec le polymère de l'invention (exemple 5-2) montre une intensité de couleur de 17 % supérieure à l'essai de l'art antérieur (exemple 5-1).

**Exemple 6**

On fabrique une composition de mascara, à partir des ingrédients indiqués dans le tableau ci-dessous. Les valeurs mentionnées dans la dernière colonne du tableau indiquent les masses en grammes.

**Tableau 5**

|  |  |  |
|---|---|---|
| A | A-1 Deionized Water | QSP 100 |
|  | A-2 Methylparaben NF (Protameen) | 0,10 |
|  | A-3 Methocel 40-202 (Dow Chemical) | 0,20 |
|  | A-4 triethanolamine (99%) | 2,80 |
|  | A-5 DL Panthenol USP (DSM) | 0,50 |
|  | A-6 Avalure UR 450 (Lubrizol) | 6,00 |
|  | A-7 PVP K-30 (ISP) | 2,00 |
|  | **A-8 Additif Polymère en l'état** | 0 ou 3,00 |
| B | B-8 Iron Oxide Black 34PC3068 (Emerald Hilton Davis) | 10,00 |

(suite)

|   |   |   |
|---|---|---|
|   | C-9 Emersol 132 (Cognis) | 5,50 |
|   | C-10 Bayberry wax (F. B. Ross) | 1,80 |
|   | C-11 Protachem GMS 450 (Protameen) | 1,70 |
| C | C-12 Beeswax white (F. B. Ross) | 4,50 |
|   | C-13 Carnauba wax n°1 | 2,70 |
|   | C-14 WW Gum Rosin (Akzo) | 1,80 |
|   | C-15 Propylparaben NF (Protameen) | 0,10 |
|   | D-16 Mirasil SM (Rhodia) | 0,10 |
|   | D-17 Lipovol WGO (Clariant) | 0,10 |
| D | D-18 Phenonip (Clariant) | 0,10 |
|   | D-19 Germaben II (ISP) | 0,50 |

Phase A (phase aqueuse) :

On place A-1 sous agitation et on chauffe à 40°C puis on dissout A-2 dans A-1. On stoppe l'agitation, puis on additionne A-3. On agite jusqu'à obtenir un milieu homogène. On additionne la triéthanolamine A-4 au milieu. On incorpore ensuite A-5, puis le polymère filmogène A-6 (6 % en poids) sous agitation. On ajoute enfin le polymère filmogène A-7 (2 % en poids) et éventuellement une solution aqueuse à 40 % de matière active du polymère A-8 (3 % en poids du polymère en l'état, soit 1,2 % en poids sec). Après homogénéisation complète, on monte la température à 75°C.

Phases B (phase non aqueuse), C & D :

On mélange tous les ingrédients de la phase C à une température de 75°C. On incorpore le pigment minéral colorant B-8 (10 % en poids). On poursuit le mélange puis on refroidit à 50°C. Toujours sous agitation, on introduit les ingrédients D-16, D-17, D-18 et D-19.
Enfin on mélange la phase aqueuse A au mélange composé des phases B, C et D. On prépare donc une formulation du type eau-dans-huile.

**Essai 6-1**

Cet essai illustre la référence, et ne met en oeuvre aucun ingrédient A-8 supplémentaire autres que ceux décrits dans le tableau.

**Essai 6-2**

Cet essai illustre l'art antérieur et met en oeuvre en tant qu'ingrédient A-8 un polymère constitué de 100 % d'acide acrylique totalement neutralisé par la soude et de masse molaire moyenne en poids égale à 4 500 g/mol.
Les polymères des essais 6-3 à 6-7 sont préparés selon un procédé similaire à celui des essais 1-1 à 1-3 de l'exemple 1.

**Essai 6-3**

Cet essai illustre l'utilisation d'un polymère hors invention. Il met en oeuvre en tant qu'ingrédient A-8 un copolymère constitué de en % en poids de chacun de ses monomères :

a) 8,14 % d'acide acrylique,
b) 2,79 d'acide méthacrylique et
c) 89,07 % d'un monomère de formule (I) :

$$R\text{-}(OP)_m\text{-}(OE)_n\text{-}R' \qquad (I)$$

- m = 15, n = 46,
- OP et OE désignent respectivement l'oxyde de propylène et l'oxyde d'éthylène,
- R désigne la fonction méthacrylate,
- R' représente l'hydrogène

totalement neutralisé par la soude (NaOH) et de masse moléculaire $M_w$ en poids égale à 1 800 000 g/mol.

**Essai 6-4**

Cet essai illustre l'invention, et met en oeuvre un copolymère constitué de, en % en poids de chacun de ses monomères :

a) 7,5 % d'acide méthacrylique et
b) 92,5 % d'un monomère de formule (I) :

$$R\text{-}(OP)_m\text{-}(OE)_n\text{-}R' \qquad (I)$$

- m = 15, n = 46,
- OP et OE désignent respectivement l'oxyde de propylène et l'oxyde d'éthylène,
- R désigne la fonction méthacrylate,
- R' représente l'hydrogène,

totalement neutralisé par la soude et de masse moléculaire $M_w$ en poids égale à 120 000 g/mol.

**Essai 6-5**

Cet essai illustre l'invention, et met en oeuvre un copolymère constitué de en % en poids de chacun de ses monomères :

a) 12,5 % d'acide méthacrylique
b) 87,5 % d'un monomère de formule (I) :

$$R\text{-}(OP)_m\text{-}(OE)_n\text{-}R' \qquad (I)$$

- m = 15, n = 46,
- OP et OE désignent respectivement l'oxyde de propylène et l'oxyde d'éthylène,
- R désigne la fonction méthacrylate,
- R' représente l'hydrogène,

totalement neutralisé par la soude et de masse moléculaire $M_w$ en poids égale à 75 000 g/mol.

**Essai 6-6**

Cet essai illustre l'invention, et met en oeuvre un copolymère constitué de en % en poids de chacun de ses monomères :

a) 12,8 % d'acide acrylique
b) 87,2 % d'un monomère de formule (I) :

$$R\text{-}(OP)_m\text{-}(OE)_n\text{-}R' \qquad (I)$$

- m = 15, n = 46,
- OP et OE désignent respectivement l'oxyde de propylène et l'oxyde d'éthylène,
- R désigne la fonction méthacrylate,
- R' représente l'hydrogène,

totalement neutralisé par la soude et de masse moléculaire $M_w$ en poids égale à 45 000 g/mol.

**Essai 6-7**

Cet essai illustre l'utilisation d'un polymère hors invention. Il met en oeuvre un copolymère constitué de en % en poids de chacun de ses monomères :

a) 5,1 % d'acide acrylique et
b) 94,9 % d'un monomère de formule (I):

$$R\text{-}(OP)_m\text{-}(OE)_n\text{-}R' \qquad (I)$$

- m = 0, n = 113,
- OE désigne l'oxyde d'éthylène,
- R désigne la fonction méthacrylate,
- R' représente le radical méthyl,

totalement neutralisé par la soude et de masse moléculaire $M_w$ en poids égale à 65 000 g/mol.

Mesure de la viscosité

Les viscosités sont mesurées avec un viscosimètre Brookfield, modèle RVT. Chacune des formulations de mascara est conditionnée dans des flacons en verre standard. Avant la mesure de la viscosité, on laisse chacune des formulations au repos 24 heures à 25 °C. Le mobile doit être centré par rapport à l'ouverture du flacon et on le plonge jusqu'à ce que la surface de la formulation affleure le repère.
On mesure ensuite la viscosité à 20 rpm. On laisse tourner le viscosimètre jusqu'à ce que la viscosité soit stable.

Elaboration des mascaras et évaluation de ses propriétés organoleptiques

Chacune des formulations est élaborée tel que décrit plus haut. L'observation des aspects organoleptiques est effectuée à température ambiante. Les critères pris en compte sont : Etalement (couvrance), Texture (lisse, présence de grumeaux, de grains, aspect « flan » ou filant), Odeur (apparition ou non d'une odeur), Couleur (variation de l'homogénéité de la couleur), Surface (lisse ou non lisse).

Stabilité des mascaras au stockage :

Chacune des formulations est conditionnée dans des flacons en verre 120 mL appropriées. Les flacons sont ensuite introduits, dans une étude à 45°C. A t = 3 mois, les flacons sont sortis de l'étuve et les potentielles instabilités suivantes sont observées : déphasage ou séparation de phase, crémage, exsudation, relargage, dépôt ou sédimentation.

**Tableau 6**

| Essai | REFerence/AA/ INVention/Hors INvention | Viscosité Brookfield (cPs, 20 rpm, 25°C) | Elaboration & Propriétés organoleptiques | Stabilité au stockage Vieillissement 3 mois à 45°C |
|---|---|---|---|---|
| 6-1 | REF | 14610 | **\* Elaboration** : Stable<br>**\* Organoleptique** :<br>Etalement : R.A.S.<br>Texture : R.A.S.<br>Odeur: R.A.S.<br>Couleur : R.A.S.<br>Surface : R.A.S.<br>**Conclusion : Stable** | Début sédimentation - Exsudation |

(suite)

| Essai | REFerence/AA/ INVention/Hors INvention | Viscosité Brookfield (cPs, 20 rpm, 25°C) | Elaboration & Propriétés organoleptiques | Stabilité au stockage Vieillissement 3 mois à 45°C |
|---|---|---|---|---|
| 6-2 | AA | Non mesurable | **\* Elaboration** : instable<br>Floculation & Déplétion<br>**\* Organoleptique** : NA<br>**Conclusion : Instable** | NA* |
| 6-3 | H-IN | 14050 | **\* Elaboration** : Stable<br>**\* Organoleptique** :<br>Etalement : R.A.S.<br>Texture : grains<br>Odeur : R.A.S.<br>Couleur : R.A.S.<br>Surface : R.A.S.<br>**Conclusion : légèrement instable** | Sédimentation - Relargage |
| 6-4 | INV | 11550 | **\* Elaboration** : Stable<br>**\* Organoleptique** :<br>Etalement : R.A.S.<br>Texture : R.A.S.<br>Odeur: R.A.S.<br>Couleur : R.A.S.<br>Surface : R.A.S.<br>**Conclusion : Stable** | Stable - Rien à signaler |
| 6-5 | INV | 9230 | **\* Elaboration** : Stable<br>**\* Organoleptique** :<br>Etalement : R.A.S.<br>Texture : R.A.S.<br>Odeur: R.A.S.<br>Couleur : R.A.S.<br>Surface : R.A.S.<br>**Conclusion : Stable** | Stable - Rien à signaler |
| 6-6 | INV | 10140 | **\* Elaboration** : Stable<br>**\* Organoleptique** :<br>Etalement : R.A.S.<br>Texture : R.A.S.<br>Odeur : R.A.S.<br>Couleur : R.A.S.<br>Surface : R.A.S.<br>**Conclusion : Stable** | Stable - Rien à signaler |

(suite)

| Essai | REFerence/AA/ INVention/Hors INvention | Viscosité Brookfield (cPs, 20 rpm, 25°C) | Elaboration & Propriétés organoleptiques | Stabilité au stockage Vieillissement 3 mois à 45°C |
|---|---|---|---|---|
| 6-7 | H-INV | 16 200 | * **Elaboration** : Stable<br>* **Organoleptique** :<br> Etalement : R.A.S.<br> Texture : grains<br> Odeur : R.A.S.<br> Couleur : R.A.S.<br> Surface : non lisse<br> **Conclusion :<br> légèrement instable** | Sédimentation - Exsudation |
| *NA : non applicable* | | | | |

Les résultats du tableau 6 démontrent que la double sélection opérée sur le choix du macromonomère d'une part, et sur la masse molaire du copolymère d'autre part, conduisent à des compositions homogènes caractérisées par une diminution de la viscosité (valeurs toutes inférieures à 14 000 cPs) qui ne présentent pas de difficultés (formation d'hétérogénéités, précipitation, déplétion...) lors de l'élaboration du mascara, ainsi qu'une stabilité au stockage.

Ainsi, on déduit un très bon niveau de compatibilité entre le polymère de l'invention et le polymère filmogène utilisé dans une formulation cosmétique qui comprend 10% en poids d'oxyde de fer.

**Exemple 7** - **Variation de la quantité de pigment minéral colorant dans la composition cosmétique**

On prépare une composition de mascara résistant à l'eau (ou waterproof), à partir des ingrédients suivants (les chiffres dans la dernière colonne indiquent les masses en grammes) :

**Tableau 7**

| | | |
|---|---|---|
| A | A-1 Beeswax (Koster Keunen Inc) | 5,00 |
| | A-2 Carnauba wax ( Koster Keunen Inc ) | 5,00 |
| | A-3 Candelilla wax ( Koster Keunen Inc ) | 3,00 |
| | A-4 Stearic Acid (Masso) | 2,00 |
| | A-5 Glyceryl Stearate (Masso) | 1,20 |
| | A-6 Isopropyl Palmitate (Stéarinerie Dubois) | 3,00 |
| | A-7 Petrolatum | 4,00 |
| | A-8 DC 245 (Dow Corning) | 3,00 |
| B | B-9 Carbopol® ETD 2050 (Lubrizol™) | 0,10 |
| | B-10 Veegum® Ultra (R. T. Vanderbilt) | 0,50 |
| | B-11 Phenonip® (Clariant) | 0,90 |
| | B-12 Iron Oxide Black 34-PV-3069 (Emerald Hilton Davis) | z |
| | B-13 Deionized Water | QSP 100 |
| | B-14 Avalure UR 450 (Lubrizol™) | 15,00 |
| | B-15 DC 193 Surfactant (Dow Corning) | 64,00 |
| | **B-16 Additif Polymère en l'état** | 3,00 |
| C | C-16 triethanolamine (99%) | q.s. pH 7.5 |

On mélange tout d'abord à température ambiante B-9, B-10, B-13 et éventuellement l'additif polymère B-16 (3 % en poids en l'état par rapport au poids total de la composition cosmétique) (800 à 1000 rpm pendant 60 minutes).

On ajoute ensuite à ce mélange, les composés B-11, B-12, le polymère filmogène B-14 (14 % en poids par rapport au poids total de la composition cosmétique), B-15. On homogénéise. On chauffe ce mélange de la phase B à 60°C.

Parallèlement, on mélange l'ensemble des ingrédients de la phase A, sauf l'ingrédient A-8 et on chauffe à 85°C. On additionne ensuite, à ce mélange, l'ingrédient A-8.

On incorpore la phase non aqueuse A dans le mélange aqueux B et on homogénéise. On compense l'eau évaporée. On ajuster le pH à 7,5 avec l'ingrédient C-16.

On refroidit sous agitation. On a ainsi préparé donc une formulation du type huile-dans-eau.

**Essai 7-1**

Cet essai illustre la référence, et ne met en oeuvre aucun ingrédient supplémentaire B-16 autres que ceux décrits dans le tableau 7.

**Essai 7-2**

Cet essai illustre l'art antérieur et met en oeuvre en tant qu'ingrédient B-16 un polymère constitué de 100 % d'acide acrylique, totalement neutralisé par la soude et de masse molaire moyenne en poids égale à 4 500 g/mol.

**Essai 7-3**

Cet essai illustre l'invention, et met en oeuvre en tant qu'ingrédient B-16 un copolymère constitué de en % en poids de chacun de ses monomères :

a) 12,5 % d'acide méthacrylique
b) 87,5 % d'un monomère de formule (I) :

$$R\text{-}(OP)_m\text{-}(OE)_n\text{-}R' \qquad (I)$$

- m = 15, n = 46,
- OP et OE désignent respectivement l'oxyde de propylène et l'oxyde d'éthylène,
- R désigne la fonction méthacrylate,
- R' représente Hydrogène,

totalement neutralisé par la soude et de masse molaire moyenne en poids égale à 75 000 g/mol.

**Tableau 8**

| Essai | Z (% massique) | Viscosité Brookfield (cPs, 20 rpm, 25°C) | Elaboration & Propriétés organoleptiques | Stabilité au stockage Vieillissement 3 mois à 45°C |
|---|---|---|---|---|
| 7-1 REF | 3 | 15 270 | **\* Elaboration :** Stable<br>**\* Organoleptique :**<br>Etalement : R.A.S.<br>Texture : R.A.S.<br>Odeur : R.A.S.<br>Couleur : R.A.S.<br>Surface : R.A.S.<br>**Conclusion : Stable** | Stable - Rien à signaler |
| | 12 | 120 310 | **\* Elaboration** : Stable<br>**\* Organoleptique** :<br>Etalement : R.A.S.<br>Texture : grains<br>Odeur: R.A.S.<br>Couleur : R.A.S.<br>Surface : R.A.S.<br>**Conclusion** :<br>**légèrement instable** | Début de sédimentation |
| | 25 | Non mesurable | NA | Non mesurable |
| 7-2 AA | 3 | Non mesurable | **\* Elaboration** :<br>instable<br>Floculation & Déplétion<br>**\* Organoleptique :**<br>**NA**<br>**Conclusion :**<br>**Instable** | NA |
| | 12 | Non mesurable | **\* Elaboration** :<br>instable<br>Floculation & Déplétion<br>**\* Organoleptique :**<br>**NA**<br>**Conclusion :**<br>**Instable** | NA |
| | 25 | Non mesurable | **\* Elaboration** :<br>instable<br>Floculation & Déplétion<br>**\* Organoleptique :**<br>**NA**<br>**Conclusion :**<br>**Instable** | NA |

(suite)

| Essai | Z (% massique) | Viscosité Brookfield (cPs, 20 rpm, 25°C) | Elaboration & Propriétés organoleptiques | Stabilité au stockage Vieillissement 3 mois à 45°C |
|---|---|---|---|---|
| 7-3 INV | 3 | 10 810 | **\* Elaboration** : Stable<br>**\* Organoleptique** :<br>Etalement : R.A.S.<br>Texture : R.A.S<br>Odeur : R.A.S.<br>Couleur : R.A.S.<br>Surface : R.A.S.<br>**Conclusion** : **Stable** | Stable - Rien à signaler |
| | 12 | 60 180 | **\* Elaboration :** Stable<br>**\* Organoleptique :**<br>Etalement : R.A.S.<br>Texture : R.A.S<br>Odeur : R.A.S.<br>Couleur : R.A.S.<br>Surface : R.A.S.<br>**Conclusion : Stable** | Stable - Rien à signaler |
| | 25 | 112 180 | **\* Elaboration** : Stable<br>**\* Organoleptique** :<br>Etalement : R.A.S.<br>Texture : R.A.S<br>Odeur : R.A.S.<br>Couleur : R.A.S.<br>Surface : R.A.S.<br>**Conclusion** : **Stable** | Stable - Rien à signaler |

Les résultats du tableau 8 démontrent que l'utilisation du copolymère de l'invention dans une formulation de mascara « waterproof », permet d'homogénéiser, de réduire la viscosité tout en présentant une stabilité lors de la mise en oeuvre et sur le test de vieillissement, et ce, y compris pour des formulations comprenant 12 et 25 % en poids d'oxydes de fer. Ainsi, on déduit un très bon niveau de compatibilité entre le polymère de l'invention et le polymère filmogène.

**Revendications**

**1.**   Utilisation d'un copolymère hydrosoluble de type peigne, constitué :

a) d'au moins un monomère anionique présentant une fonction vinylique polymérisable et un groupement carboxyle et
b) d'au moins un macromonomère de formule (I) :

$$R\text{-}(OP)_m\text{-}(OE)_n\text{-}R' \qquad (I)$$

dans laquelle :

m et n sont des entiers non nuls et inférieurs ou égaux à 150,
OP et OE désignent respectivement l'oxyde de propylène et l'oxyde d'éthylène, et sont disposés de manière aléatoire ou de manière régulière,
R désigne une fonction insaturée polymérisable,
R' représente l'hydrogène ou un groupement alkyle ayant de 1 à 4 atomes de carbone,

ledit copolymère présentant une masse moléculaire comprise entre 20 000 g/mol et 400 000 g/mol déterminée par chromatographie par perméation de gel, pour homogénéiser les compositions cosmétiques pour le maquillage et/ou le soin des matières kératiniques comprenant un polymère filmogène et un pigment colorant et/ou une nacre.

2. Utilisation selon la revendication 1, dans laquelle le copolymère est constitué :

a) d'au moins un monomère acide (méth)acrylique et
b) d'au moins un macromonomère de formule (I) :

$$R\text{-}(OP)_m\text{-}(OE)_n\text{-}R' \qquad (I)$$

dans laquelle :

m et n sont des entiers non nuls et inférieurs à 150,
OP et OE désignent respectivement l'oxyde de propylène et l'oxyde d'éthylène, et sont disposés de manière aléatoire ou de manière régulière,
R désigne une fonction insaturée polymérisable,
R' représente l'hydrogène ou un groupement alkyle ayant de 1 à 4 atomes de carbone,

ledit copolymère présentant une masse moléculaire comprise entre 20 000 g/mol et 250 000 g/mol.

3. Utilisation selon la revendication 1 ou 2, selon laquelle ledit pigment colorant est choisi dans le groupe consistant en les oxydes de fer, les oxydes de chrome, les oxydes de zirconium, de zinc ou de cérium, le violet de manganèse, le bleu outremer, le noir de carbone, l'hydrate de chrome et le bleu ferrique.

4. Utilisation selon l'une quelconque des revendications précédentes, selon laquelle le copolymère est constitué, exprimé en pourcentage en poids de chacun de ses constituants, de :

a) 5 % à 30 % d'au moins un monomère acide (méth)acrylique,
b) 70 % à 95 % d'au moins un macromonomère de formule (I),
c) 0 % à 20 % d'au moins un monomère qui est un ester de l'acide (méth)acrylique,

la somme des pourcentages a), b) et c) étant égale à 100 %.

5. Utilisation selon l'une quelconque des revendications précédentes, selon laquelle le copolymère est constitué, exprimé en pourcentage en poids de chacun de ses constituants, de :

a) 5 % à 15 % d'au moins un monomère acide (méth)acrylique,
b) 85 % à 95 % d'au moins un macromonomère de formule (I),
c) 0 % à 10 % d'au moins un monomère qui est un ester de l'acide (méth)acrylique,

la somme des pourcentages a), b) et c) étant égale à 100 %.

6. Utilisation selon l'une quelconque des revendications précédentes, selon laquelle le copolymère (méth)acrylique peigne présente une masse moléculaire comprise entre 30 000 g/mol et 380 000 g/mol, par exemple comprise entre 30 000 g/mol et 150 000 g/mol.

7. Utilisation selon l'une quelconque des revendications précédentes, selon laquelle ladite composition cosmétique comprend de 3 à 25 % en poids de pigments colorants et/ou de nacre et de 8 à 15 % en poids de polymère filmogène.

8. Utilisation selon l'une quelconque des revendications précédentes, selon laquelle, m et n sont compris entre 10 et 90 dans la formule (I).

9. Composition cosmétique pour le maquillage et/ou le soin des matières kératiniques, comprenant :

a) de 1 à 30 % en poids de pigments colorants et/ou de nacre,
b) de 0,1 à 30 % en poids d'un polymère filmogène, et

c) de 0,05 à 8 % en poids d'un copolymère hydrosoluble de type peigne constitué :

c1) d'au moins un monomère anionique présentant une fonction vinylique polymérisable et un groupement carboxyle et

c2) d'au moins un macromonomère de formule (I) :

$$R\text{-}(OP)_m\text{-}(OE)_n\text{-}R' \qquad (I)$$

dans laquelle :

m et n sont deux entiers non nuls et inférieurs à 150,
OP et OE désignent respectivement l'oxyde de propylène et l'oxyde d'éthylène, et sont disposés de manière aléatoire ou de manière régulière,
R désigne une fonction insaturée polymérisable,
R' représente l'hydrogène ou un groupement alkyle ayant de 1 à 4 atomes de carbone,

ledit copolymère présentant une masse moléculaire comprise entre 20 000 g/mol et 400 000 g/mol.

10. Composition cosmétique selon la revendication 9, comprenant :

a) de 1 à 30 % en poids de pigment minéral colorant,
b) de 0,1 à 30 % en poids d'un polymère filmogène, et
c) de 0,5 à 8 % en poids d'un copolymère (méth)acrylique peigne constitué :

c1) d'au moins un monomère acide (méth)acrylique et
c2) d'au moins un macromonomère de formule (I) :

$$R\text{-}(OP)_m\text{-}(OE)_n\text{-}R' \qquad (I)$$

dans laquelle :

m et n sont deux entiers non nuls et inférieurs à 150,
OP et OE désignent respectivement l'oxyde de propylène et l'oxyde d'éthylène, et sont disposés de manière aléatoire ou de manière régulière,
R désigne une fonction insaturée polymérisable,
R' représente l'hydrogène ou un groupement alkyle ayant de 1 à 4 atomes de carbone,

ledit copolymère présentant une masse moléculaire comprise entre 20 000 g/mol et 250 000 g/mol.

11. Composition cosmétique selon la revendication 9 ou 10, comprenant de 1 à 80 % en poids de cire ou d'huile, volatile ou non.

12. Composition cosmétique selon l'une quelconque des revendications 9 à 11, comprenant de 2 à 5 % en poids dudit copolymère.

13. Composition cosmétique selon l'une quelconque des revendications 9 à 12, comprenant de 0,1 à 15 % en poids d'un agent épaississant de phase aqueuse.

14. Composition cosmétique selon l'une quelconque des revendications 9 à 13, comprenant de 0,5 à 20 % en poids d'un polymère filmogène.

15. Composition cosmétique selon l'une quelconque des revendications 9 à 14, comprenant au moins un autre additif choisi dans le groupe consistant en des particules réfléchissantes, des agents tensioactifs ou émulsionnants ou émulsifiants, des émollients, des humectants, des hydratants, des agents neutralisants, des agents alcalinisants, des agents acidifiants, des conservateurs, des charges, des antioxydants, des parfums, des plastifiants, des actifs cosmétiques et des filtres solaires.

**Patentansprüche**

1. Verwendung eines wasserlöslichen kammartigen Copolymers bestehend aus:

   a) mindestens einem anionischen Monomer mit einer polymerisierbaren funktionellen Vinylgruppe und einer Carboxylgruppe sowie
   b) mindestens einem Makromonomer mit der Formel (I):

   $$R\text{-}(OP)_m\text{-}(OE)_n\text{-}R' \qquad (I)$$

   worin:

   m und n Ganzzahlen ungleich Null von weniger oder gleich 150 sind,
   OP und OE jeweils Propylenoxid und Ethylenoxid entsprechen und entweder willkürlich oder regelmäßig angeordnet sind,
   R eine polymerisierbare ungesättigte funktionelle Gruppe ist,
   R' Wasserstoff oder eine Alkylgruppe mit 1 bis 4 Kohlenstoffatomen ist,

   wobei das Copolymer eine Molmasse zwischen 20.000 g/mol und 400.000 g/mol, gemessen mittels Gelpermeationschromatographie, aufweist, um kosmetische Zusammensetzungen für das Make-up und/oder die Pflege von Keratinmaterialien, umfassend ein filmbildendes Polymer und ein Farbpigment und/oder ein Perlglanzmittel, zu homogenisieren.

2. Verwendung nach Anspruch 1, worin das Copolymer besteht aus:

   a) mindestens einem (Meth)acrylsäure-Monomer und
   b) mindestens einem Makromonomer mit der Formel (I):

   $$R\text{-}(OP)_m\text{-}(OE)_n\text{-}R' \qquad (I)$$

   worin:

   m und n Ganzzahlen ungleich Null von weniger oder gleich 150 sind,
   OP und OE jeweils Propylenoxid und Ethylenoxid entsprechen und entweder willkürlich oder regelmäßig angeordnet sind,
   R eine polymerisierbare ungesättigte funktionelle Gruppe ist,
   R' Wasserstoff oder eine Alkylgruppe mit 1 bis 4 Kohlenstoffatomen ist,

   wobei das Copolymer eine Molmasse zwischen 20.000 g/mol und 250.000 g/mol aufweist.

3. Verwendung nach Anspruch 1 oder 2, bei der dieses Farbpigment aus der Gruppe bestehend aus Eisenoxiden, Chromoxiden, Zirkonium-, Zink- oder Ceroxiden, Manganviolett, Ultramarinblau, Rußschwarz, hydratisiertem Chrom und Eisenblau ausgewählt ist.

4. Verwendung nach einem beliebigen der vorstehenden Ansprüche, worin das Copolymer, in Gewichtsprozent seiner Bestandteile ausgedrückt, besteht aus:

   a) zu 5 bis 30 % aus mindestens einem (Meth)acrylsäure-Monomer,
   b) zu 70 bis 95 % aus mindestens einem Makromonomer mit der Formel (I),
   c) zu 0 bis 20 % aus mindestens einem Monomer, das ein Ester der (Meth)acrylsäure ist,

   wobei die Summe der Prozentanteile von a), b) und c) gleich 100 % ist.

5. Verwendung nach einem beliebigen der vorstehenden Ansprüche, worin das Copolymer, in Gewichtsprozent seiner Bestandteile ausgedrückt, besteht aus:

   a) zu 5 bis 15 % aus mindestens einem (Meth)acrylsäure-Monomer,
   b) zu 85 bis 95 % aus mindestens einem Makromonomer mit der Formel (I),

c) zu 0 bis 10 % aus mindestens einem Monomer, das ein Ester der (Meth)acrylsäure ist,

wobei die Summe der Prozentanteile von a), b) und c) gleich 100 % ist.

6. Verwendung nach einem beliebigen der vorstehenden Ansprüche, bei der das kammartige (Meth)acrylsäure-Copolymer eine Molmasse zwischen 30.000 g/mol und 380.000 g/mol, beispielsweise zwischen 30.000 g/mol und 150.000 g/mol, aufweist.

7. Verwendung nach einem beliebigen der vorstehenden Ansprüche, bei der diese kosmetische Zusammensetzung 3 bis 25 Gew.-% Farbpigmente und/oder Perlglanzmittel und 8 bis 15 Gew.-% eines filmbildenden Polymers umfasst.

8. Verwendung nach einem beliebigen der vorstehenden Ansprüche, bei der m und n in der Formel (I) zwischen 10 und 90 liegen.

9. Kosmetische Zusammensetzung für das Makeup und/oder die Pflege von Keratinmaterialien, umfassend:

a) 1 bis 30 Gew.-% Farbpigmente und/oder Perlglanzmittel,
b) zu 0,1 bis 30 Gew.-% ein filmbildendes Polymer, und
c) 0,05 bis 8 Gew.-% eines wasserlöslichen kammartigen Copolymers, bestehend aus:

c1) mindestens einem anionischen Monomer mit einer polymerisierbaren funktionellen Vinylgruppe und einer Carboxylgruppe sowie
c2) mindestens einem Makromonomer mit der Formel (I):

$$R\text{-}(OP)_m\text{-}(OE)_n\text{-}R' \qquad (I)$$

worin:

m und n Ganzzahlen ungleich Null von weniger oder gleich 150 sind,
OP und OE jeweils Propylenoxid und Ethylenoxid entsprechen und entweder willkürlich oder regelmäßig angeordnet sind,
R eine polymerisierbare ungesättigte funktionelle Gruppe ist,
R' Wasserstoff oder eine Alkylgruppe mit 1 bis 4 Kohlenstoffatomen ist,

wobei das Copolymer eine Molmasse zwischen 20.000 g/mol und 400.000 g/mol aufweist.

10. Kosmetische Zusammensetzung nach Anspruch 9, umfassend:

a) zu 1 bis 30 Gew.-% mineralische Farbpigmente,
b) zu 0,1 bis 30 Gew.-% ein filmbildendes Polymer, und
c) zu 0,5 bis 8 Gew.-% ein kammartiges (Meth)acrylsäure-Copolymer, bestehend aus:

c1) mindestens einem (Meth)acrylsäure-Monomer und
c2) mindestens einem Makromonomer mit der Formel (I):

$$R\text{-}(OP)_m\text{-}(OE)_n\text{-}R' \qquad (I)$$

worin:

m und n Ganzzahlen ungleich Null von weniger oder gleich 150 sind,
OP und OE jeweils Propylenoxid und Ethylenoxid entsprechen und entweder willkürlich oder regelmäßig angeordnet sind,
R eine polymerisierbare ungesättigte funktionelle Gruppe ist,
R' Wasserstoff oder eine Alkylgruppe mit 1 bis 4 Kohlenstoffatomen ist,

wobei das Copolymer eine Molmasse zwischen 20.000 g/mol und 250.000 g/mol aufweist.

**11.** Kosmetische Zusammensetzung nach Anspruch 9 oder 10, umfassend zu 1 bis 80 Gew.-% Wachs oder Öl, das flüchtig oder nicht flüchtig sein kann.

**12.** Kosmetische Zusammensetzung nach einem beliebigen der vorstehenden Ansprüche 9 bis 11, umfassend zu 2 bis 5 Gew.-% dieses Copolymer.

**13.** Kosmetische Zusammensetzung nach einem beliebigen der vorstehenden Ansprüche 9 bis 12, umfassend zu 0,1 bis 15 Gew.-% ein Verdickungsmittel für die wässrige Phase.

**14.** Kosmetische Zusammensetzung nach einem beliebigen der vorstehenden Ansprüche 9 bis 13, umfassend zu 0,5 bis 20 Gew.-% ein filmbildendes Polymer.

**15.** Kosmetische Zusammensetzung nach einem beliebigen der vorstehenden Ansprüche 9 bis 14, umfassend mindestens ein anderes Additiv, das aus der Gruppe bestehend aus lichtreflektierenden Teilchen, Tensiden oder Emulgatoren oder Emulsionsmitteln, Weichungsmitteln, Feuchthaltemitteln, Feuchtigkeitsspendern, Neutralisierungsmitteln, Alkalisierungsmitteln, Säuerungsmitteln, Konservierungsmitteln, Füllmitteln, Antioxidationsmitteln, Duftstoffen, Weichmachern, kosmetischen Wirkstoffen und Sonnenschutzfiltern ausgewählt ist.

## Claims

**1.** Use of a water-soluble comb-type copolymer, made up of:

a) at least one anionic monomer having a polymerizable vinyl group and a carboxyl group, and
b) at least one macro-monomer of formula (I):

$$R\text{-}(PO)_m\text{-}(EO)_n\text{-}R' \qquad (I)$$

wherein:

m and n are integers other than zero and less than or equal to 150,
PO and EO designate respectively propylene oxide and ethylene oxide, and are arranged either randomly or regularly,
R designates a polymerizable unsaturated group,
R' is hydrogen or an alkyl group having 1 to 4 carbon atoms,

said copolymer having a molecular mass between 20,000 and 400,000 g/mol determined by gel permeation chromatography, to homogenize cosmetic compositions for makeup and/or for the care of keratinous materials comprising a film-forming polymer and a coloring pigment and/or a pearlescent.

**2.** Use according to claim 1, wherein the copolymer is made up of:

a) at least one monomer of (meth)acrylic acid and
b) at least one macro-monomer of formula (I):

$$R\text{-}(PO)_m\text{-}(EO)_n\text{-}R' \qquad (I)$$

wherein:

m and n are integers other than zero and lower than 150,
PO and EO designate respectively propylene oxide and ethylene oxide, and are arranged either randomly or regularly,
R designates a polymerizable unsaturated group,
R' is hydrogen or an alkyl group having 1 to 4 carbon atoms,

the molecular mass of said copolymer being between 20,000 g/mol and 250,000 g/mol.

**3.** Use according to claim 1 or 2, in which said coloring pigment is chosen from the group that includes iron oxides, chromium oxides, zirconium oxides, zinc or cerium oxides, manganese purple, blue ultramarine, carbon black, chromium hydrate and iron blue.

4. Use according to any one of the previous claims, in which the copolymer is made up of, expressing the percentage by weight of each of its constituents:

a) 5% to 30% of at least one monomer of (meth)acrylic acid,
b) 70% to 95% of at least one macromonomer of formula (I),
c) 0% to 20% of at least one monomer which is an ester of (meth)acrylic acid,

the sum of the percentages a), b) and c) is equal to 100%.

5. Use according to any one of the previous claims, in which the copolymer is made up of, expressing the percentage by weight of each of its constituents:

a) 5% to 15% of at least one monomer of (meth)acrylic acid,
b) 85% to 95% of at least one macromonomer of formula (I),
c) 0% to 10% of at least one monomer which is an ester of (meth)acrylic acid,

the sum of the percentages a), b) and c) is equal to 100%.

6. Use according to any of the previous claims, in which the comb-type (meth)acrylic copolymer has a molecular mass between 30,000 and 380,000 g/mol, for example between 30,000 g/mol and 150,000 g/mol.

7. Use according to any one of the previous claims, whereby said cosmetic composition comprises 3 to 25% by weight of coloring pigments and/or pearlescent and 8 to 15% by weight of film-forming polymer.

8. Use according to any one of the previous claims, wherein m and n are between 10 and 90 in the formula (I).

9. Cosmetic composition for makeup and/or for the care of keratinous materials, comprising:

a) from 1 to 30% by weight of coloring pigment and/or pearlescent,
b) from 0.1 to 30% by weight of film-forming polymer, and
c) from 0.05 to 8% by weight of a comb-type water-soluble copolymer made up of:

c1) at least one anionic monomer having a polymerizable vinyl group and a carboxyl group, and
c2) at least one macro-monomer of formula (I):

$$R\text{-}(PO)_m\text{-}(EO)_n\text{-}R' \qquad (I)$$

wherein:

m and n are integers other than zero and lower than 150,
PO and EO designate respectively propylene oxide and ethylene oxide, and are arranged either randomly or regularly,
R designates a polymerizable unsaturated group,
R' is hydrogen or an alkyl group having 1 to 4 carbon atoms,

the molecular mass of said copolymer being between 20,000 g/mol and 400,000 g/mol.

10. Cosmetic composition according to claim 9, comprising:

a) from 1 to 30% by weight of coloring mineral pigment,
b) from 0.1 to 30% by weight of film-forming polymer, and
c) between 0.5 to 8% by weight of comb-type (meth)acrylic copolymer made up of:

c1) at least one monomer of (meth)acrylic acid and
c2) at least one macro-monomer having the formula (I):

$$R\text{-}(PO)_m\text{-}(EO)_n\text{-}R' \qquad (I)$$

wherein:

    m and n are integers other than zero and lower than 150,
    PO and EO designate respectively propylene oxide and ethylene oxide, and are arranged either randomly or regularly,
    R designates a polymerizable unsaturated group,
    R' is hydrogen or an alkyl group having 1 to 4 carbon atoms,

the molecular mass of said copolymer being between 20,000 g/mol and 250,000 g/mol.

11. Cosmetic composition according to the claim 9 or 10, comprising 1 to 80% by weight of wax or oil, volatile or non-volatile.

12. Cosmetic composition according to any one of the claims 9 to 11, comprising 2 to 5% by weight of said copolymer.

13. Cosmetic composition according to any one of the claims 9 to 12, comprising 0.1% to 15% by weight of an aqueous-phase thickening agent.

14. Cosmetic composition according to anyone of the claims 9 to 13, comprising 0.5% to 20% by weight of a film-forming polymer.

15. Cosmetic composition according to anyone of the claims 9 to 14, comprising at least one other additive chosen from the group consisting of reflective particles, surfactants or emulsifying agents or emulsifiers, emollients, humectants, hydrating agents, neutralizing, alkalizing, acidifying agents, preservatives, fillers, antioxidants, perfumes, plasticizers, cosmetic active ingredients and sunscreens.

**RÉFÉRENCES CITÉES DANS LA DESCRIPTION**

**Documents brevets cités dans la description**

- US 20120076840 A
- JP 2012001503 B
- WO 2009073384 A

**Littérature non-brevet citée dans la description**

- *Abstract of Paper, 244th ACS National Meeting & Exposition,* 19 Août 2012
- *J. Cosmet. Sci.,* 1999, vol. 50, 105-109
- *Tenside Surf. Det.,* 1999, vol. 36